# FASCICULE DE BREVET EUROPEEN

(11) **EP 1 121 108 B1**
(45) Date de publication et mention de la délivrance du brevet: **02.04.2003**
(21) Numéro de dépôt: 99946255.9
(22) Date de dépôt: 29.09.1999
(51) Int. Cl.: A61K 31/135, A61P 15/06

(54) **NOUVELLE UTILISATION THERAPEUTIQUE DE COMPOSES A ACTIVITE BETA-3-AGONISTE**
NEUE THERAPEUTISCHE ANWENDUNG VON VERBINDUNGEN MIT BETA-3-AGONISTISCHER AKTIVITÄT
NOVEL THERAPEUTIC USE OF COMPOUNDS WITH BETA-3-AGONIST ACTIVITY

(30) Priorité: 14.10.1998 FR 9812877
(43) Date de publication de la demande: 08.08.2001
(73) Titulaire: SANOFI-SYNTHELABO, 75013 Paris (FR)
(72) Inventeur: ADVENIER, Charles, Fac. de Médec. de Paris Ouest, 75006 Paris (FR); MANARA, Luciano, I-15040 Pietra Morazzi (IT)
(74) Mandataire: Varady, Peter
(86) Numéro de dépôt international: FR9902308
(87) Numéro de publication internationale: WO00021508

(56) Documents cités:
- EP-A- 0 626 367
- BARDOU MARC, ET AL.: "In vitro inhibition of human colonic motility with SR 59119A and SR 59104A: Evidence of a beta-3-adrenoceptor-mediated effect" EUR. J. PHARMACOL., vol. 353, no. 2/3, 1998, pages 281-287, XP000872299
- MANARA LUCIANO, ET AL.: "Functional identification of rat atypical beta-3-adrenoceptors by the first beta-3-selective antagonists, aroyloxypropanolaminotetralins" BR. J. PHARMACOL., vol. 117, no. 3, 1996, pages 435-442, XP000872295

## Description

La présente invention a pour objet une nouvelle utilisation des composés actifs sur le récepteur β₃-adrénergique. Plus particulièrement, l'invention se rapporte à l'utilisation des β₃-agonistes pour la préparation de médicaments inhibant les contractions utérines.

Le récepteur β₃-adrénergique, indiqué aussi parfois comme récepteur β atypique est principalement localisé dans le tissu adipeux et dans le tractus gastro-intestinal.

De nombreuses demandes de brevet ont été publiées ces dernières années décrivant de nouveaux produits ayant activité β₃-agoniste.

Les produits sont indiqués dans plusieurs pathologies telles que l'obésité, le diabète, l'hyperglycémie, ou les désordres de l'appareil gastro-intestinal. Jusqu'à présent aucune demande de brevet ou publication scientifique n'a envisagé la possibilité d'utiliser des composés ayant une activité β₃-agoniste en tant qu'inhibiteurs de la contraction utérine, afin d'obtenir un effet bénéfique en cas de dysménorrhée ou une activité tocolytique.

Les produits normalement utilisés en tant qu'agents tocolytiques sont les agonistes β₂ et notamment la ritodrine et le salbutamol.

On sait qu'à cause de l'effet au niveau pulmonaire et cardiaque causé par tous les β₂-agonistes, l'utilisation de la ritodrine ou du salbutamol entraîne souvent des effets secondaires importants.

Pour le traitement de la dysménorrhée, les produits normalement utilisés sont les anti-inflammatoires non-stéroïdiens, qui ont des effets secondaires, surtout au niveau gastrique, bien connus.

Les composés β₃-adrénergiques ont été testés initialement chez le rat et ce n'est que ces dernières années que les chercheurs ont mis au point des essais pour évaluer l'activité de ces composés dans les tissus humains.

Croci et al. rapportent que des β₃ agonistes sélectifs tels que la N-[(6-hydroxy-1,2,3,4-tétrahydronapht-2-yl)méthyl]-2-hydroxy-2-(3-chorophényl)éthanamine et la N-[7-méthoxy-1,2,3,4-tétrahydronapht-2-yl)méthyl]-2-hydroxy-2-(3-chorophényl)éthanamine sont inactifs sur l'utérus du rat (Br.J.Pharmacol., 116, Proc. Suppl. 204 P, 1995).

De la même façon, Landi et al. montrent que la N-[(2S)-7-carboxy-méthoxy-1,2,3,4-tétrahydronapht-2-yl]-(2R)-2-(3-chorophényl)-2-hydroxyéthanamine, β₃- agoniste sélective, n'a pas d'activité sur l'utérus du rat. (Br. J. Pharmacol. 114, Proc. Suppl., 432P, 1995).

L'activité que certains β₃-agonistes présentent sur l'utérus de rat est en effet à attribuer à leur faible sélectivité vis-à-vis des récepteur β₂. (J. Pharmacol. Exp. Ther. 277, 1, 22-27, 1996).

Nous avons maintenant trouvé, de façon tout à fait surprenante, que les β₃- agonistes causent un effet relaxant des muscles de l'utérus humain.

Ainsi, selon l'un de ses aspects, la présente invention a pour objet l'utilisation d'un β₃-agoniste sélectif pour la préparation d'un médicament inhibant les contractions utérines chez l'être humain.

Plus particulièrement, l'invention a pour objet l'utilisation d'un β₃-agoniste sélectif pour la préparation d'un médicament destiné à traiter la dysménorrhée ou ayant un effet tocolytique.

Les β₃-agonistes objet de la présente invention sont par exemple les produits compris dans les brevets ou demandes de brevets suivants: EP 436 435, EP 500 443, WO 98/20005, JP 10007647, US 5,705,515, EP 822 185, WO 97/46556, WO 97/43273, JP 09268171, FR 2746395, WO 97/37646, EP 801 060, WO 97/34905, WO 97/25311, WO 97/21666, WO 97/21665, JP 09118655, WO 97/15549, GB 2305665, EP 764 640, EP 764 632, WO 96/35671, WO 96/35685, JP 08259558, US 5,561,142, JP 08198866, JP 08165276, JP 08157470, EP 714 883, WO 96/16938, WO 96/04234, WO 96/04233, US 5,488,064, US 5,491,134, US 5,482,971, WO 95/29159, WO 95/33724, ZA 9409874, JP 07228543, WO 95/25104, EP 659 737, WO 95/11223, WO 95/08527, WO 95/07284, JP 07112958, WO 95/04047, JP 06345731, WO 94/29290, JP 06293664, WO 94/24090, EP 611 003, EP 608 568, WO 94/12166, US 5,321,036, WO 93/22277, WO 94/02493, EP 565 317, WO 93/15041, WO 94/16938.

Des β₃-agonistes avantageux selon la présente invention sont représentés par la formule (I): 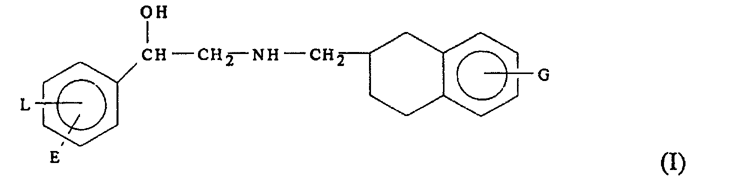 dans laquelle
- E: représente un groupe l'hydrogène, un groupe (C₁-C₄)alkyle, un groupe (C₁-C₄)alkoxy, un groupe phényle, un groupe nitro, un atome d'halogène ou un groupe trifluorométhyle,
- L: représente un groupe l'hydrogène, un groupe (C₁-C₄)alkyle, un groupe (C₁-C₄)alkoxy, un groupe phényle, un groupe nitro, un atome d'halogène ou
- E et L ,: ensemble, représentent un groupe -CH=CH-CH=CH- ou CH₂-CH₂-CH₂-CH₂-, et
- G: représente l'hydrogène, un atome de chlore, un groupe hydroxy ou un groupe OG' où G' représente un groupe (C₁-C₄)alkyle non substitué ou substitué par un groupe hydroxy, (C₁-C₄)alkoxy, (C₁-C₄)alkoxycarbonyle, carboxy ou (C₃-C₇)cycloalkyle; un groupe (C₃-C₇)cycloalkyl; un groupe (C₂-C₄)alcanoyle,
ainsi que leurs sels pharmaceutiquement acceptables, décrits dans EP 0 436 435 comme comme spasmolytiques intestinaux.

Parmi les composés de formule (I), la N-[(6-hydroxy-1,2,3,4-tétrahydronaphtalen-(2R)-2-yl)méthyl]-(2R)-2-hydroxy-2-(3-chlorophényl)éthanamine, la N-[(7-méthoxy-1,2,3,4-tétrahydronaphtalen-(2R))-2-yl)méthyl]-(2R)-2-hydroxy-2-(3-chlorophényl)éthanamine, ainsi que leurs sels pharmaceutiquement acceptables, sont des composés particulièrement avantageux.

D'autres β₃-agonistes avantageux selon la présente invention sont les composés suivants:
- le produit L-755507 décrit dans EP 611 003 de formule (a): 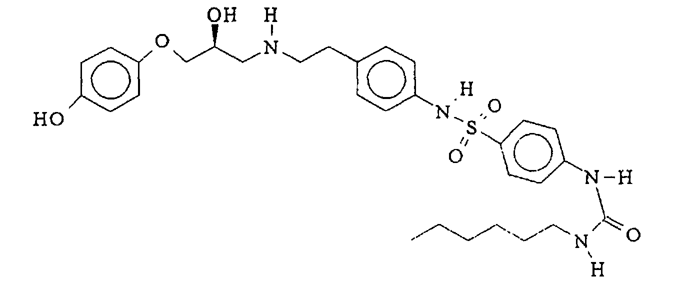
- le produit L-750355 décrit dans EP 611 003 de formule (b): 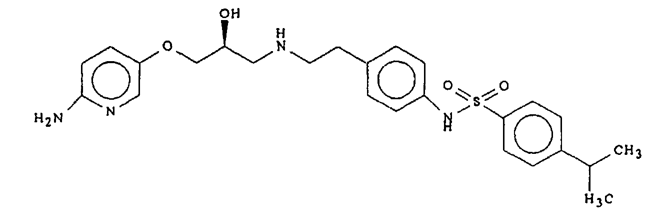
- le produit L-759574 décrit dans EP 611 003 de formule (c): 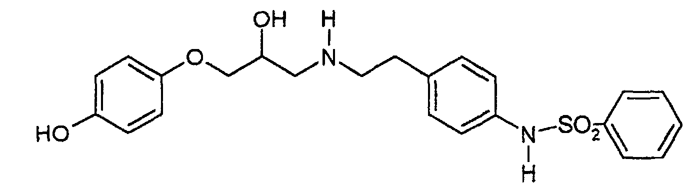
- le produit décrit dans WO 95/29159 de formule (d): 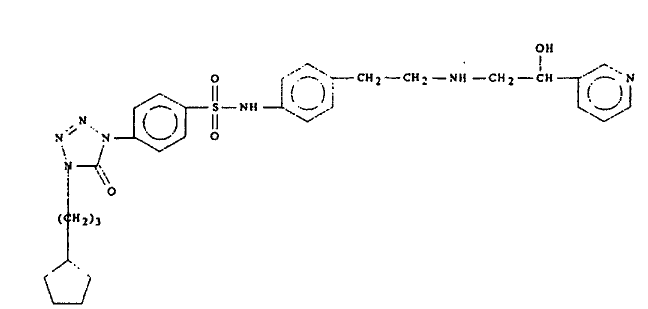
- le produit décrit dans WO 95/0047 de formule (e): 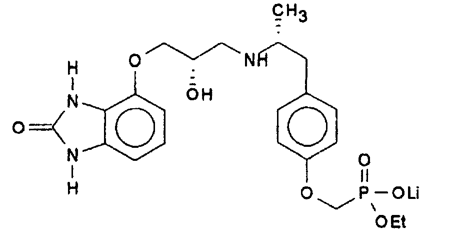
- le produit décrit dans WO 96/04233de formule (f): 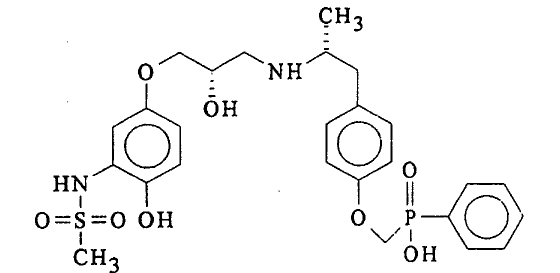
- le produit SB-226552 décrit dans WO 96/04233 de formule (g): 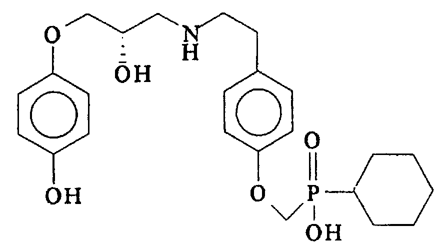
- le produit décrit dans EP 764 640 de formule (h): 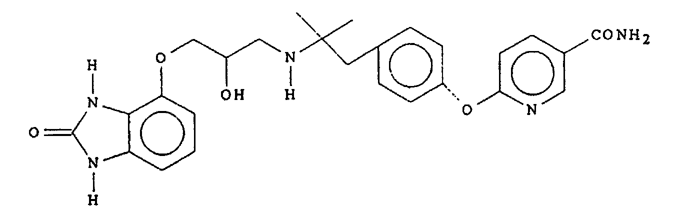
- le produit CP-331679 décrit dans WO 94/29290de formule (i): 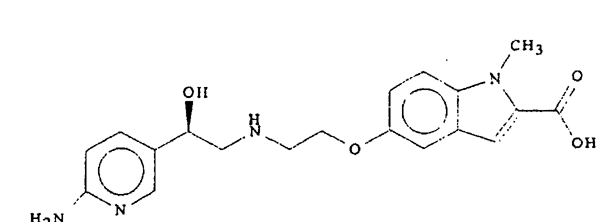
- le produit décrit dans EP 659 737 de formule (j): 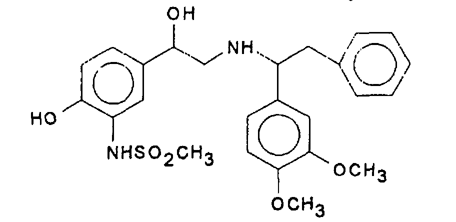
- le produit BMS 187257 décrit dan US 5,321,036 de formule (k): 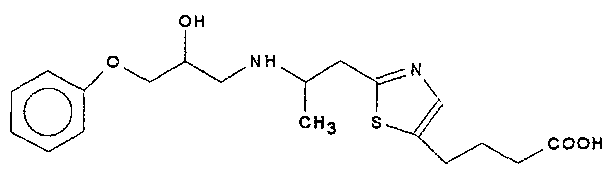
- le produit AD-9677 décrit dans WO 94/16938 de formule (1): 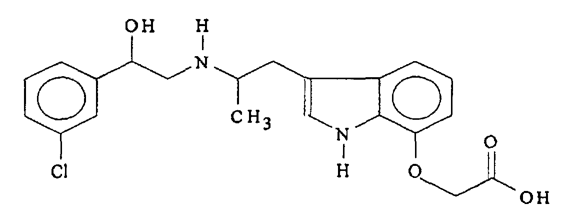
- le produit FR-149175 décrit dans WO 93/15041de formule (m):

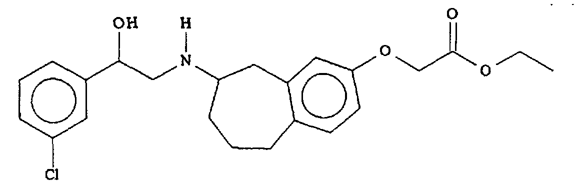

Selon un autre de ses aspects, la présente invention a pour objet l'inhibition des contractions utérines, par l'utilisation d'une dose efficace d'un composé β₃-agoniste.

L'activité des composés a été mise en évidence en mesurant l'inhibition des contractions spontanées sur des échantillons d'utérus humain.

Les β₃-agonistes testés, notamment la N-[(6-hydroxy-1,2,3,4-tétrahydronapht-2-yl)méthyl]-2-hydroxy-2-(3-chorophényl)éthanamine et la N-[7-méthoxy-1,2,3,4-tétrahydronapht-2-yl)méthyl]-2-hydroxy-2-(3-chorophényl)éthanamine, ont montré une capacité d'inhibition des contractions de l'utérus similaire à celle du salbutamol, un produit ayant une activité β₂-agoniste, qui exerce un effet tocolytique grâce à cette activité (Fundam. Clin. Pharmacol., 1995, 9 (4): 407).

En outre, il a été vérifié que l'inhibition de la contraction utérine provoquée par les composés de l'invention n'est pas bloquée par l'ajout d'un β₂-agoniste tel que le propranolol ce qui démontre que cette activité tocolytique ne peut être attribuée à une action sur le récepteur β₂

### Essais d'inhibition des contractions utérines

### Préparation de tissus du myomètre humain

Des tissus du myomètre ont été obtenus de femmes à la grossesse normale presque à terme sans complications (entre la 38ème et la 40 semaines de gestation) mais devant être soumises à l'opération césarienne. Des échantillons de tissus ont été excisés de la couche longitudinale du corps de l'utérus, sur le site antiplacentaire, et placés immédiatement en solution de Krebs à 4°C (composition en mM: NaCl 118, KCl 5.4, CaCl₂ 2.5, KH₂PO₄ 0.6, MgSO₄ 1.2, NaHCO₃ 25, glucose 11.7). Les tissus ont été disséqués sans membrane séreuse et utilisés frais. L'utilisation du tissu du myomètre humain pour des tests a été approuvé par le comité éthique local.

### Etude fonctionnelle

Des tissus du myomètre de chaque morceau de muscle ont été coupés en 6-8 bandes (de 8-10 mm de long et 2-3 mm de section) et suspendus de façon isométrique sous une tension de repos de 2 g dans un bain pour organes de 20 ml contenant une solution de Krebs (composition comme ci-dessus) à 37°C et aérés à 95% d'O₂ et 5% de CO₂ (ph 7.40). Après une heure, pendant laquelle les bandes du myomètre ont été lavées tous les quarts d'heure et la tension de repos réglée à 2 g, les bandes ont été laissées s'équilibrer pendant une heure de plus jusqu'au moment où elles ont montré une activité contractile, rhytmique, spontanée régulière. Les changements de tension ont été mesurés au moyen de jauges de déformation de Pioden (en anglais: Pioden strain gauges) (UF1), amplifiés (EMKA; France) et enregistrés (Linseis L65514; Allemagne). Une fois l'amplitude des contractions devenue régulière, les courbes cumulatives d'inhibition concentration/réponse (de 10⁻⁸ à 3 x 10⁻⁵ M) ont été déterminées pour chaque composé étudié: salbutamol, la N-[(6-hydroxy-1,2,3,4-tétrahydronapht-2-yl)méthyl]-2-hydroxy-2-(3-chorophényl)éthanamine et la N-[7-méthoxy-1,2,3,4-tétrahydronapht-2-yl)méthyl]-2-hydroxy-2-(3-chorophényl)éthanamine. Chaque médicament a été testé sur 2-24 préparations, chacune provenant d'un patient différent. Des essais de contrôle ont été menés en utilisant du solvant. L'effet de chaque agent de relaxation a été exprimé comme un pourcentage dell'inhibition maximale obtenue en ajoutant à la fin de l'essai de la théophylline (3 x 10⁻³ M).

Dans l'essai ci-dessus, les composés N-[(6-hydroxy-1,2,3,4-tétrahydronapht-2-yl)méthyl]-2-hydroxy-2-(3-chorophényl)éthanamine et N-[7-méthoxy-1,2,3,4-tétrahydronapht-2-yl)méthyl]-2-hydroxy-2-(3-chorophényl)éthanamine ont montré une activité équivalente à celle du salbutamol.

Grâce à cet effet sur l'utérus humain, les composés de l'invention peuvent être avantageusement utilisés dans la préparation de médicaments aptes à prévenir ou retarder les accouchements précoces ou bien pour ralentir ou arrêter les accouchements pendant un bref laps de temps, suffisant pour prêter d'autres formes de soins.

De plus, les composés de l'invention peuvent bien être utilisés pour la prévention et/ou le traitement de la dysménorrhée.

Les composés β₃-agonistes pour l'utilisation selon l'invention sont administrables en compositions pharmaceutiques, préparées selon les méthodes usuelles telles que décrites par exemple dans les brevets cités ci-dessus.

Afin d'obtenir l'effet thérapeutique désiré, la dose de principe actif à administrer peut varier entre 0,01 et 100 mg par Kg de poids du corps et par jour selon la gravité des symptômes.

Chaque dose unitaire peut contenir de 0,1 à 500 mg de principe actif, préférablement en combinaison avec un support pharmaceutique. Cette dose unitaire peut être administrée 1 à 4 fois par jour.

## Revendications

1. Utilisation d'un composé à action β₃-agoniste pour la préparation d'un médicament destiné à inhiber les contractions utérines.

2. Utilisation selon la revendication 1 **caractérisée en ce que** le médicament est utilisé comme tocolytique pour prévenir ou retarder les accouchements précoces.

3. Utilisation selon la revendication 1 **caractérisée en ce que** le médicament est destiné au traitement et/ou à la prophylaxie de la dysménorrhée.

4. Utilisation selon la revendication 1 **caractérisée en ce que** ledit β₃-agoniste est un composé de formule (I) 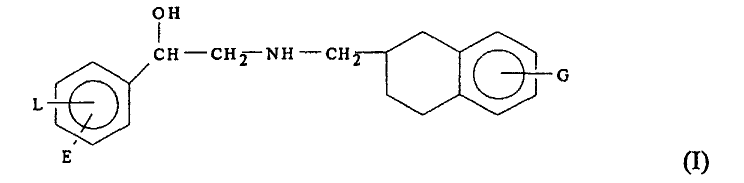 dans laquelle
E représente un groupe l'hydrogène, un groupe (C₁-C₄)alkyle, un groupe (C₁-C₄)alkoxy, un groupe phényle, un groupe nitro, un atome d'halogène ou un groupe trifluorométhyle,
L représente un groupe l'hydrogène, un groupe (C₁-C₄)alkyle, un groupe (C₁-C₄)alkoxy, un groupe phényle, un groupe nitro, un atome d'halogène ou
E et L, ensemble, représentent un groupe -CH=CH-CH=CH- ou CH₂-CH₂-CH₂-CH₂-, et
G représente l'hydrogène, un atome de chlore, un groupe hydroxy ou un groupe OG' où G' représente un groupe (C₁-C₄)alkyle non substitué ou substitué par un groupe hydroxy, (C₁-C₄)alkoxy, (C₁-C₄)alkoxycarbonyle, carboxy ou (C₃-C₇)cycloalkyle; un groupe (C₃-C₇)cycloalkyl; un groupe (C₂-C₄)alcanoyle
ou l'un de ses sels pharmacetiquement acceptables.

5. Utilisation selon la revendication 4 **caractérisée en ce que** ledit β₃-agoniste est choisi parmi la N-[(6-hydroxy-1,2,3,4-tétrahydro-naphtalen-(2R)-2-yl)méthyl]-(2R)-2-hydroxy-2-(3-chlorophényl)éthanamine et la N-[(7-méthoxy-1,2,3,4-tétrahydro-naphtalen-(2R)-2-yl)méthyl]-2R)-2-hydroxy-2-(3-chlorophényl)éthanamine ainsi que leurs sels pharmaceutiquement acceptables.

6. Utilisation selon la revendication 1 à 3 **caractérisée en ce que** ledit β₃-agoniste est choisi parmi les produits de formule (a) à (m) suivants:

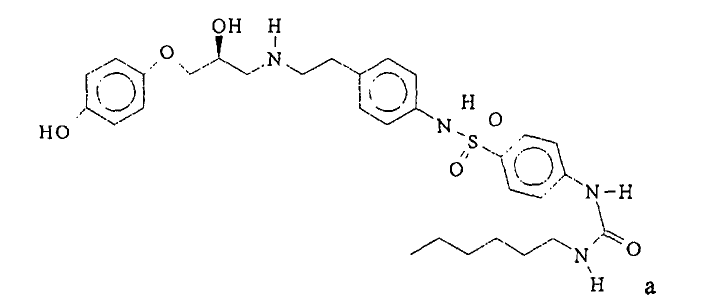

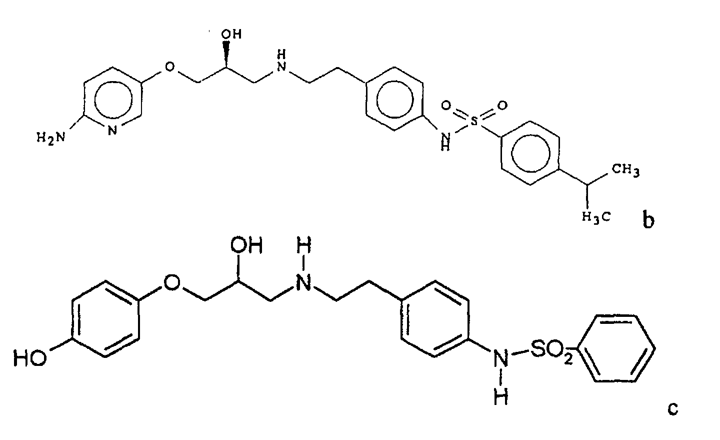

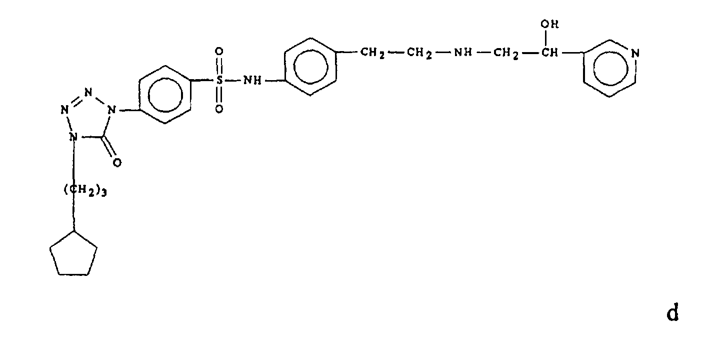

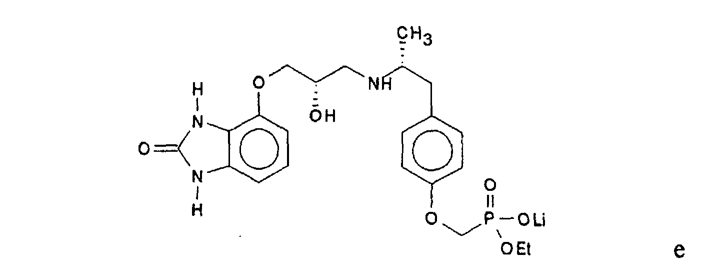

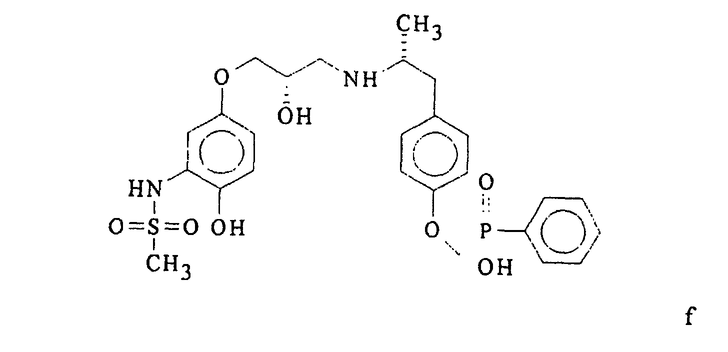

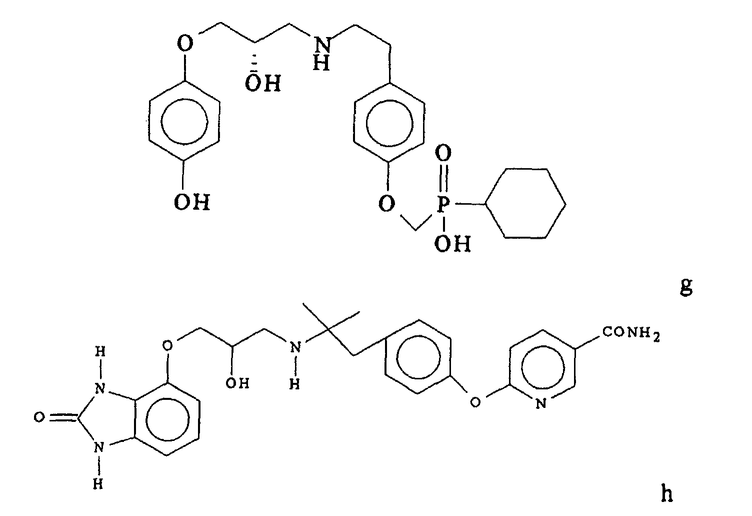

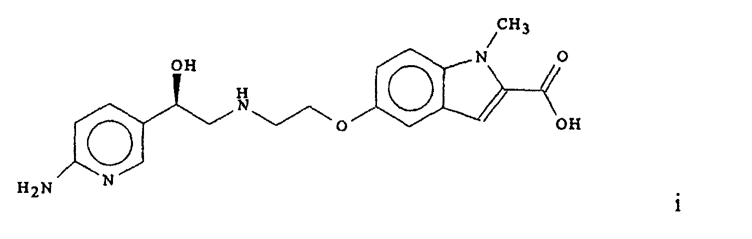

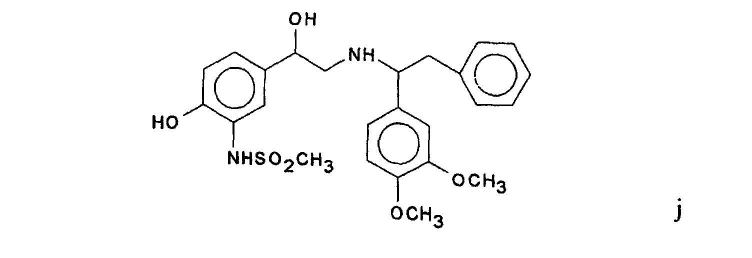

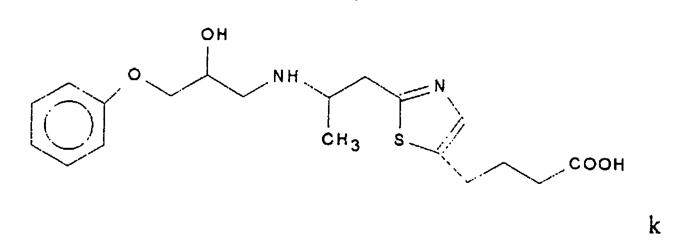

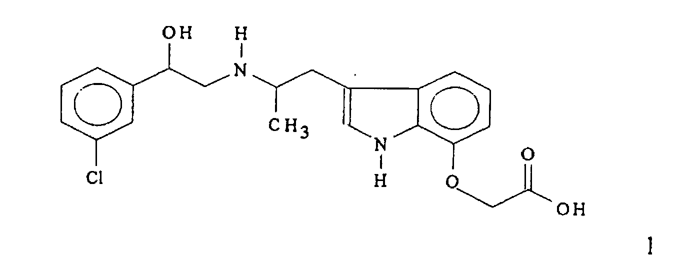

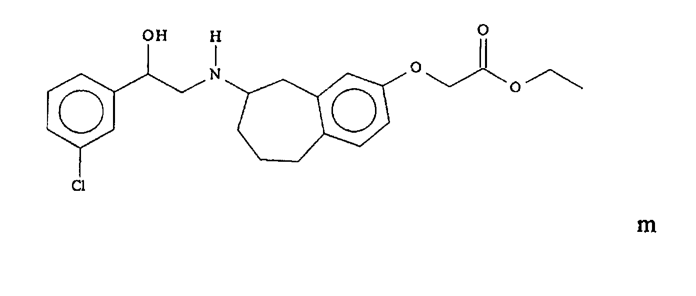

## Claims

1. Use of a compound with a β₃-agonist action in the preparation of a medicament intended to inhibit uterine contractions.

2. Use according to Claim 1, **characterized in that** the medicament is used as tocolytic agent in preventing or slowing down premature labour.

3. Use according to Claim 1, **characterized in that** the medicament is intended for the treatment and/or the prophylaxis of dysmenorrhea.

4. Use according to Claim 1, **characterized in that** the said β₃-agonist is a compound of formula (I) 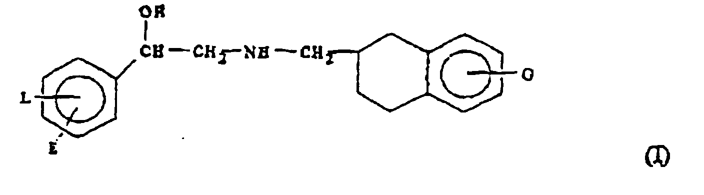 in which
E represents hydrogen, a (C₁-C₄)alkyl group, a (C₁-C₄)alkoxy group, a phenyl group, a nitro group, a halogen atom or a trifluoromethyl group,
L represents hydrogen, a (C₁-C₄)alkyl group, a (C₁-C₄)alkoxy group, a phenyl group, a nitro group or a halogen atom, or
E and L together represent a -CH=CH-CH=CH- or -CH₂-CH₂-CH₂-CH₂- group, and
G represents hydrogen, a chlorine atom, a hydroxyl group or an OG' group where G' represents a (C₁-C₄)alkyl group which is unsubstituted or substituted by a hydroxyl, (C₁-C₄)alkoxy, (C₁-C₄)alkoxycarbonyl, carboxyl or (C₃-C₇)cycloalkyl group; a (C₃-C₇)cycloalkyl group; or a (C₂-C₄)alkanoyl group,
or one of its pharmaceutically acceptable salts.

5. Use according to Claim 4, **characterized in that** the said β₃-agonist is chosen from N-[(6-hydroxy-1,2,3,4-tetrahydronaphth-(2R)-2-yl)methyl]-(2R)-2-hydroxy-2-(3-chlorophenyl)ethanamine and N-[(7-methoxy-1,2,3,4-tetrahydronaphth-(2R)-2-yl)-methyl]-(2R)-2-hydroxy-2-(3-chlorophenyl)ethan-amine and their pharmaceutically acceptable salts.

6. Use according to Claims 1 to 3, **characterized in that** the said β₃-agonist is chosen from the following products of formula (a) to (m):

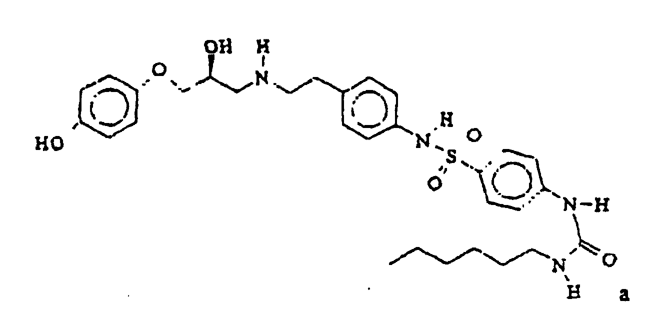

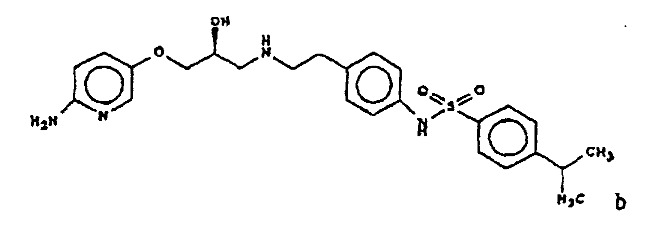

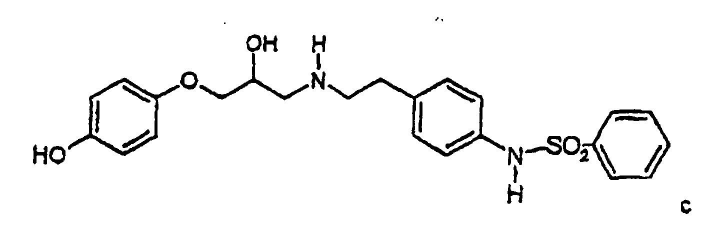

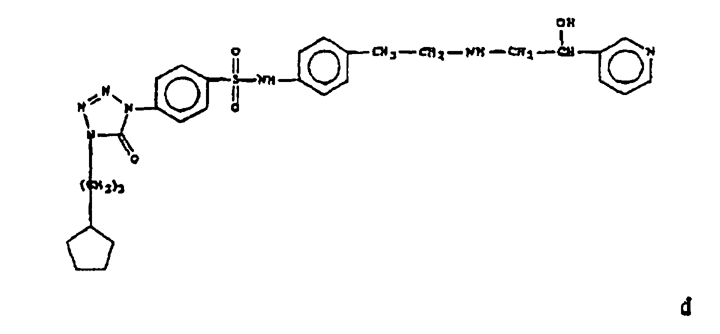

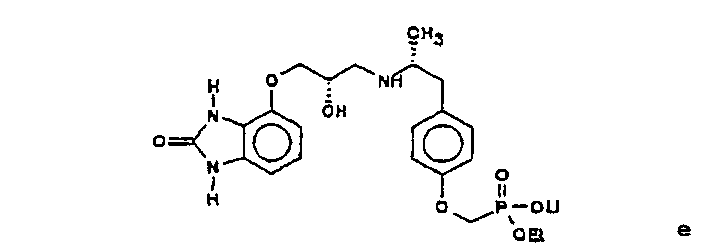

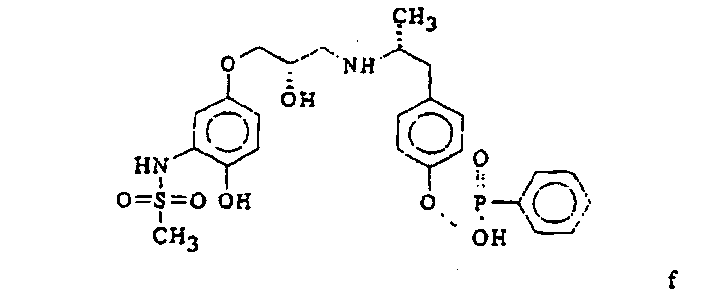

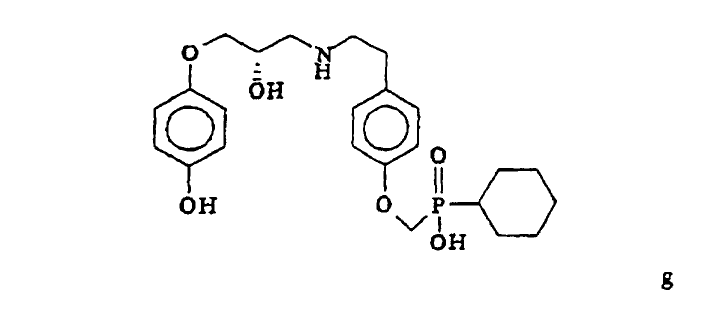

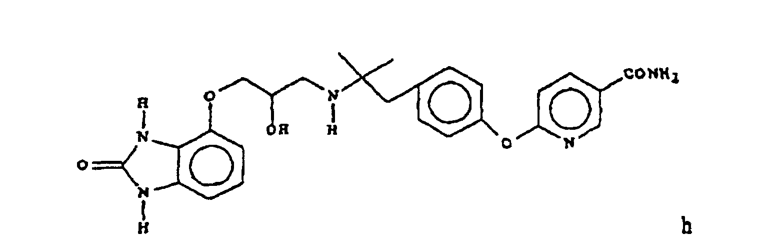

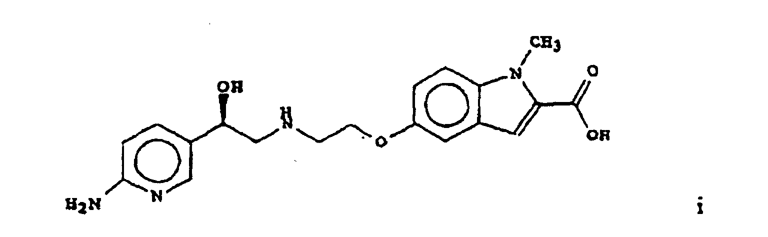

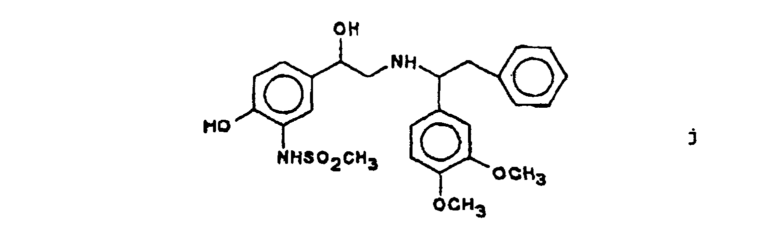

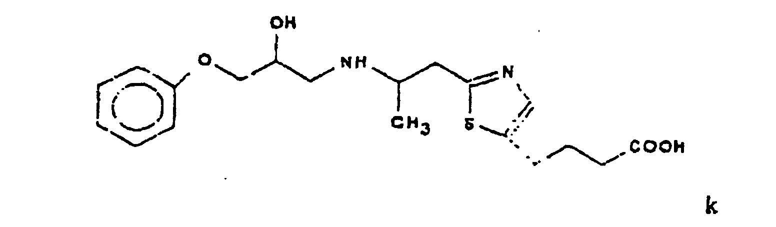

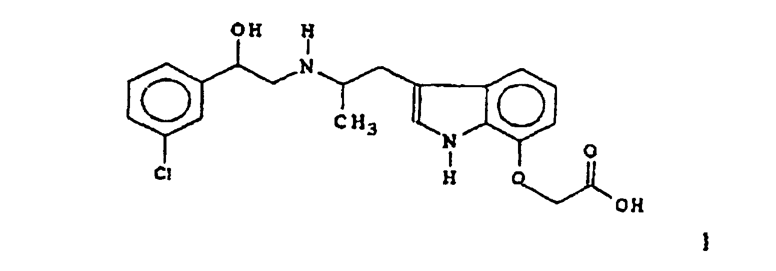

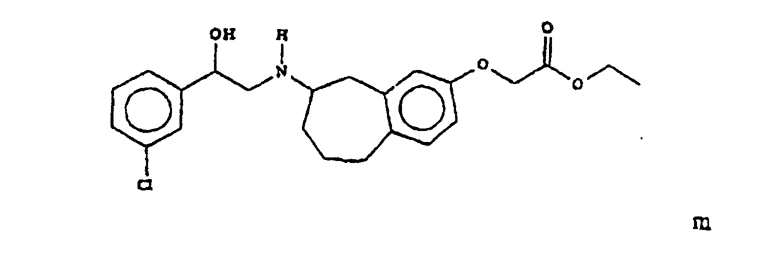

## Patentansprüche

1. Verwendung einer Verbindung mit β₃-agonistischer Wirkung für die Herstellung eines Arzneimittels zur Inhibierung von Uteruskontraktionen.

2. Verwendung nach Anspruch 1, **dadurch gekennzeichnet, daß** das Arzneimittels als Tokolytikum zur Vorbeugung und Verzögerung von Frühgeburten verwendet wird.

3. Verwendung nach Anspruch 1, **dadurch gekennzeichnet, daß** das Arzneimittel zur Behandlung und/oder der Prophylaxe der Dysmenorrhoe bestimmt ist.

4. Verwendung nach Anspruch 1, **dadurch gekennzeichnet, daß** der β₃-Agonist eine Verbindung der Formel (I) ist 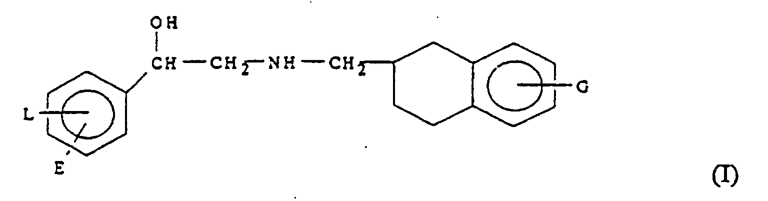 in der
E ein Wasserstoffatom, eine (C₁-C₄)-Alkylgruppe, eine (C₁-C₄)-Alkoxygruppe, eine Phenylgruppe, eine Nitrogruppe. ein Halogenatom oder eine Trifluormethylgruppe darstellt,
L ein Wasserstoffatom, eine (C₁-C₄)-Alkylgruppe, eine (C₁-C₄)-Alkoxygruppe, eine Phenylgruppe, eine Nitrogruppe oder ein Halogenatom bedeutet oder
E und L gemeinsam eine Gruppe -CH=CH-CH=CH- oder CH₂-CH₂-CH₂-CH₂- bedeuten. und
G ein Wasserstoffatom, ein Chloratom, eine Hydroxygruppe oder eine Gruppe OG' bedeutet, worin G' eine nichtsubstituierte oder durch eine Hydroxygruppe, (C₁-C₄)-Alkoxygruppe. (C₁-C₄)-Alkoxycarbonylgruppe, Carboxygruppe oder (C₃-C₇)-Cycloalkylgruppe substituierte (C₁-C₄)-Alkylgruppe; eine (C₃-C₇)-Cycloalkylgruppe oder eine (C₂-C₄)-Alkanoylgruppe bedeutet.
oder eines seiner pharmazeutisch annehmbaren Salze.

5. Verwendung nach Anspruch 4. **dadurch gekennzeichnet. daß** der β₃-Agonist ausgewählt ist aus N-[(6-Hydroxy-1,2,3,4-tetrahydro-naphthalin-(2R)-2yl)-methyl]-(2R)-2-hydroxy-2-(3-chlorphenyl)-ethanamin und N-[(7-Methoxy-1,2,3,4-tetrahydro-naphthalin-(2R)-2-yl)-methyl]-2R)-2-hydroxy-2-(3-chlorphenyl)-ethanamin und deren pharmazeutisch annehmbaren Salzen.

6. Verwendung nach Anspruch 1 bis 3, **dadurch gekennzeichnet, daß** der b₃-Agonist ausgewählt ist aus den Produkten der folgenden Formeln (a) bis (m):
